# EUROPÄISCHE PATENTANMELDUNG

(11) **EP 1 260 173 A2**
(43) Veröffentlichungstag der Anmeldung: **27.11.2002**
(21) Anmeldenummer: 02010610.0
(22) Anmeldetag: 10.05.2002
(51) Int. Cl.: A61B 5/00

(54) **Medizinisches System zur Überwachung von Parametern eines Patienten in häuslicher Umgebung**

(30) Priorität: 23.05.2001 DE 10125550
(71) Anmelder: SIEMENS AKTIENGESELLSCHAFT, 80333 München (DE)
(72) Erfinder: Tilo Christ, 91058 Erlangen (DE); Peter Leupold, 90482 Nürnberg (DE); Volker Schmidt, Dr., 91054 Erlangen (DE); Werner Striebel, 90592 Schwarzenbruck (DE)

(57) **Zusammenfassung**

Die Erfindung betrifft ein medizinisches System zur kontinuierlichen Überwachung von alterspsychiatrischen Patienten in häuslicher Umgebung oder einem Heim mit einer Vorrichtung (2) zur Erfassung von Ereignissen bei dem Patienten, einer Vorrichtung (3 bis 8) zur Übertragung der erfassten Daten der Ereignisse an eine Systemzentrale (6), und einer Empfangsvorrichtung (20 bis 22) bei einer überwachenden Person, wobei die Systemzentrale (6) einen Speicher (15) für die Daten der erfassten Ereignisse und vorgegebenen unerwünschten Ereignissen, eine Auswertevorrichtung (12) für die Daten mit einem Komparator zum Vergleich der Daten mit den gespeicherten Sollwerten der vorgegebenen unerwünschten Ereignisse, eine Alarmvorrichtung zur Erzeugung eines Alarmsignals und eine Routingvorrichtung (14) zur Weiterleitung des Alarmsignals an eine Empfangsvorrichtung (3 bis 5, 20 bis 22) der zu alarmierenden Person einer Prozesskette aufweist.

## Beschreibung

Die Erfindung betrifft ein System zur kontinuierlichen Überwachung von alterspsychiatrischen Patienten in häuslicher Umgebung, in einem Alten- oder einem Pflegeheim. Die Erfindung betrifft im Wesentlichen die Betreuung von gerontopsychiatrischen Patienten. Typische Anwendungsgebiete für ein derartiges System sind beispielsweise gerontopsychiatrische Patienten mit seniler Demenz oder hirnorganischer Psychosyndrome (HOPS).

Ein medizinisches Problem ist die Betreuung alterspsychiatrischer Patienten zu Hause, in Alten- und Pflegeheimen im Rahmen von Visiten durch den Arzt oder durch Pflegekräfte. Ziel muss es sein, laufend zu erkennen, bei welchen Patienten medizinischer Handlungsbedarf besteht, um effizient und mit guter Qualität genau die Patienten aufzusuchen, bei denen eine Intervention von Seiten des Arztes oder der Pflege erforderlich ist.

Typische Krankheitsbilder sind beispielsweise senile Demenz und hirnorganische Psychosyndrome (HOPS). An der Betreuung gerontopsychiatrischer Patienten sind viele Stellen beteiligt, die zeitlich und örtlich unabhängig voneinander handeln. Da ist zuerst der Patient, der zu betreuen ist. Dies kann bei ihm zu Hause, aber auch in einer Institution, wie beispielsweise dem Krankenhaus, Alten- oder Pflegeheim sein. Betreut wird er z.B. von Angehörigen, ambulanten Pflegedienste, Altenpfleger, Krankenschwestern, niedergelassene Ärzten, oder Klinik-Ärzten.

Durch die Vielzahl der Stellen ist die Kommunikation erschwert, so dass medizinische Befunde, die eine Reaktion erforderlich machen, in der Kommunikationskette verloren gehen oder (zu) spät beim Arzt eintreffen.

Eine Vorauswahl, zu welchen Patienten ein ambulanter Pflegedienst geht, erfolgt gelegentlich durch den Einsatz einer Online Video Verbindung zum Patienten nach Hause, über die z.T. auch Überwachungsmaßnahmen, wie beispielsweise das Kleben von EKG Elektroden durch den Patienten zu Hause, durchgeführt werden.

Die Erfindung geht von der Aufgabe aus, ein System der eingangs genannten Art zu schaffen, das auf einfache Weise eine Überwachung und Beurteilung des Zustandes eines alterspsychiatrischen Patienten bei räumlicher Trennung von Arzt oder Pflegediensten und eine gezielte Information interessierter Parteien, der Ärzte, Pfleger, Patienten und/oder Kliniken ermöglicht.

Die Aufgabe wird erfindungsgemäß durch die Merkmale des Patentanspruches 1 gelöst. Dieses erfindungsgemäße System ermöglicht ein Home Monitoring anhand "unerwünschter Ereignisse" wie beispielsweise Verwirrtheitszustände, Stürze, Aggressivität, Vagabundieren, zu starke Sedierung oder Medikamentennebenwirkungen. Dadurch erhält man beispielsweise eine "virtuelle" Pflegestation beim Patienten zu Hause.

Ein solches System kommt zum Einsatz, um beispielsweise medizinische Leistungen, die einfache Überwachungsmaßnahmen erfordern, aus dem Krankenhausbereich oder der Arztpraxis herauszulösen und in die Lebensumgebung des Patienten oder ein Pflegeheim zu verlagern. Insbesondere werden von dem System drei Größen überwacht: Die Patientencompliance, die eigentlichen unerwünschter Ereignisse und die Arztcompliance. Damit können Alarme ausgelöst werden, wenn der Patient Verwirrtheitszustände, Aggressivität, Medikamentennebenwirkungen oder zu starke Sedierung zeigt, Stürze oder Vagabundieren auftreten und wenn die zu alarmierende Stelle nicht adäquat reagiert.

Erforderliche Sofortmaßnahmen im Krankheitsfalle lassen sich unverzüglich einleiten, wenn die Alarmvorrichtung derart ausgebildet ist, dass sie ein Alarmsignal bei Überschreitung insbesondere bei signifikanter Überschreitung der Daten von gespeicherten Sollwertgrenzen erzeugt, das an eine Empfangsvorrichtung einer Person einer Behandlungskette bzw. Notfallkette geleitet wird.

Erfindungsgemäß kann die Alarmvorrichtung derart ausgebildet sein, dass sie ein Alarmsignal bei Ausbleiben von Daten erzeugt, das an eine Empfangsvorrichtung des Patienten geleitet wird, und/oder dass sie ein Alarmsignal bei Ausbleiben von Reaktionen der Behandlungskette auf besonders gekennzeichnete Werte erzeugt, das an eine Empfangsvorrichtung der Notfallkette geleitet wird.

Es hat sich als vorteilhaft erwiesen, wenn die Routingvorrichtung derart ausgebildet ist, dass das Alarmsignal an eine vorbestimmte, auswählbare Empfangsvorrichtung der Prozesskette geleitet wird oder dass das Alarmsignal durch automatisches Routing an eine von der Routingvorrichtung unter Berücksichtigung der Verfügbarkeit bestimmte Empfangsvorrichtung der Prozesskette geleitet wird.

Eine schnelle und effektive Benachrichtigung im Notfall lässt sich erreichen, wenn die Routingvorrichtung ein lernendes Expertensystem aufweist, das die Alarmierung der Prozesskette, beispielsweise die Verständigung des Hausarztes, Rufen des Notarztes, Organisieren des Transportdienstes und/oder Vorbereitung der Klinik, bewirkt.

Unerwünschte Fehlmeldungen werden reduziert bzw. ausgeschlossen, wenn die Auswertevorrichtung ein lernendes Expertensystem aufweist, das die Daten krankheits- und/oder problemspezifisch anhand von Regelsystemen oder auf Wahrscheinlichkeiten basierend interpretiert, aufgrund deren Ergebnis das Alarmsignal ausgelöst wird.

Die Erfindung ist nachfolgend anhand von in der Zeichnung dargestellten Ausführungsbeispielen näher erläutert.

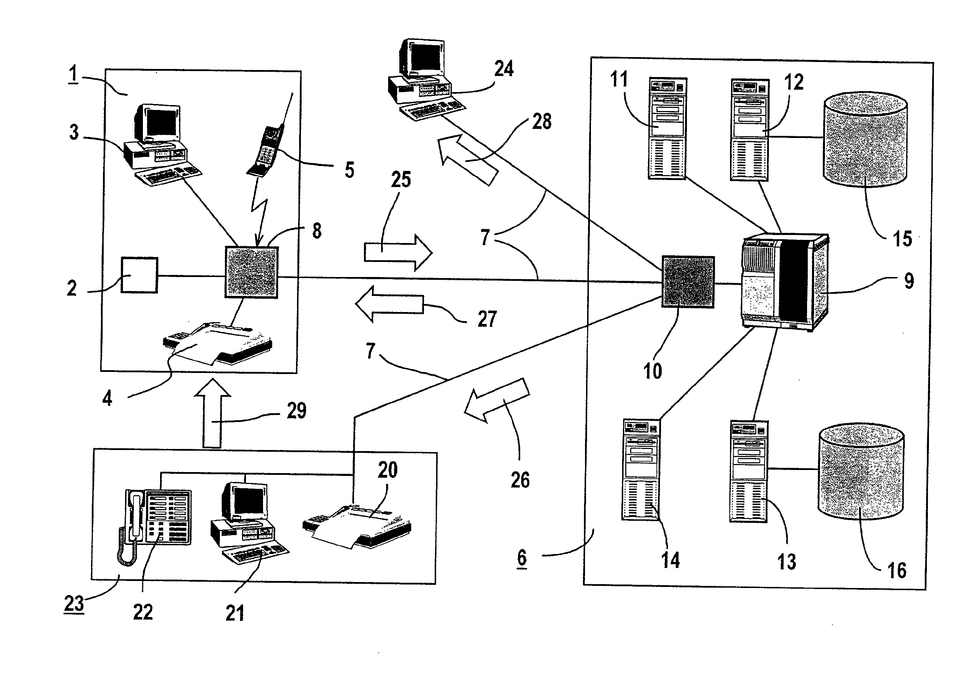

In der Figur 1 ist ein erfindungsgemäßes System zur kontinuierlichen Überwachung unerwünschter Ereignisse bei einem Patienten in seinem Haus 1 dargestellt, in dem die Erfassung von unerwünschten Ereignissen entweder automatisch beispielsweise durch einen Detektor 2 oder manuell durch den Patienten oder eine Pflegeperson erfolgt. Als Detektor können die in der DE 196 37 383 A oder der WO 99/06979 A beschriebenen Erfassungsvorrichtungen dienen. Diese erfassten Ereignisse können direkt oder per Personal Computer 3, per Faxgerät 4 oder per Telefon oder Handy 5 an eine Systemzentrale 6 weitergeleitet werden. Dies kann beispielsweise über ein ISDN-Netz 7 erfolgen, an das die entsprechenden Endgeräte über ein ISDN-Interface 8 angeschlossen sind.

In der Systemzentrale 6 ist ein Gateway 9 vorgesehen, das über ein ISDN-Interface 10 an dem ISDN-Netz 7 angeschlossen. An dem Gateway 9 kann ein Internet-Proxy-Server 11 zum Zugriff auf das Internet, eine Auswertevorrichtung 12 für die Ereignisse, ein Patientendaten-Server 13 zur Verwaltung der Patientendaten und ein Kommunikations-Server 14 als Routingvorrichtung zur Zusammenarbeit aller Komponenten und Weiterleitung von Meldungen angeschlossen sein. Mit der Auswertevorrichtung 12 ist ein Datenspeicher 15 für die Ereignisdaten und mit dem Patientendaten-Server 13 eine Datenbank 16 als Speichervorrichtung verbunden.

Die Auswertevorrichtung 12 weist einen Komparator zum Vergleich des Auftretens von Ereignissen mit in dem Datenspeicher 15 gespeicherten Sollwertgrenzen und eine Alarmvorrichtung zur Erzeugung eines Alarmsignals bei Überschreitung der Anzahl der Ereignisse von gespeicherten Sollwertgrenzen auf. Dabei kann die Auswertevorrichtung 12 die verschiedenen Ereignisse unterschiedlich bewerten.

An der Systemzentrale 6 sind Empfangsgeräte wie beispielsweise ein Faxgerät 20, Personal Computer 21 oder ein Telefon 22 oder Handy angeschlossen, die zu einer Ärzte-Bereitschaft oder eines Pflegedienstes gehören und beispielsweise in einer Gemeinschaftspraxis 23 von Ärzten angeordnet sein kann.

Weiterhin ist ein Personal Computer 24 einer Praxis eines erstbehandelnden Arztes über das ISDN-Netz 7 mit dem Gateway 9 der Systemzentrale 6 verbunden.

In der Systemzentrale 6 werden die medizinischen Daten über genormte Telekommunikationsschnittstellen eingelesen und in dem Datenspeicher 15 langfristig abgespeichert, die Auswertevorrichtung 12 wertet die unerwünschten Ereignisse und den Zeitpunkt der Erfassung aus, um Informationsnachrichten versenden zu können. Die Datenübertragung erfolgt beispielsweise über das Internet oder Telefonnetz. Die Vergabe einer Patientenidentifikationsnummer erfolgt über eine Sicherheitsarchitektur.

In der Systemzentrale 6 können alle Informationen zusammengeführt werden, die zur Realisierung der speziellen Ausführungen des Alarmgebers und zur Realisierung einer Weiterleitung von Nachrichten bei Abwesenheit des Empfängers benötigt werden.

Die Endgeräte 2 bis 5 für den Patienten dienen zur Erfassung der unerwünschten Ereignisse beim Patienten und Übertragung der Daten an die Systemzentrale 6. Die Datenübergabe 25 der Ereignisdaten an den Server in der Systemzentrale 6 kann durch den Detektor 2 selbst erfolgen, oder über unten beschriebene Eingabemöglichkeiten realisiert werden.

Für die Pfleger ist ein Personal Digital Assistant (PDA) oder ein Laptop vorgesehen, in dem alle Patientendaten eingegeben werden, die von diesem Pflegedienst besucht werden. Die Synchronisierung der Daten kann sofort, beispielsweise per Handy-Internet Verbindung, oder zu einem späteren Zeitpunkt erfolgen, wenn die Schwester wieder im Büro oder Praxis eintrifft.

Die Systemzentrale 6 dient zur Entgegennahme medizinischer Daten über eine der Telekommunikationsschnittstellen und Speicherung in dem Datenspeicher 15. Weiterhin soll sie eine Weiterverarbeitung der medizinischen Daten mit Hilfe der Auswertevorrichtung 12 für die unerwünschten Ereignisse mit der Alarmvorrichtung und dem Datenspeicher 15 bewirken. Eine Ausgabe der im Datenspeicher 15 gewonnenen Daten an eines der Endgeräte für Ärzte erfolgt über eine Telekommunikationsschnittstelle.

Ein Alarm wird durch die Alarmvorrichtung in der Auswertevorrichtung 12 bei Vorliegen einer der Ereigniskonstellation wie das Ausbleiben von Daten, unerwünschten Ereignissen oder Ausbleiben der Reaktion auf generierte Alarme ausgelöst. Dabei wird ein Alarm 26 aufgrund des Überschreitens der Anzahl der unerwünschten Ereignisse von den Sollwerten an die Endgeräte 20 bis 22 der Gemeinschaftspraxis 23 weitergeleitet. Ein Alarm 27 an den Patienten wird wegen fehlender Daten ausgelöst. In der Praxis kann der Arzt die in dem Datenspeicher 15 gespeicherten Daten 28 mit dem Personal Computer 24 abgerufen werden.

Die Auswertevorrichtung 12 beurteilt, ob die unerwünschten Ereignisse oder deren Anzahl als auffällig im Hinblick auf den gerontopsychiatrischen Patienten einzustufen sind. Als Kriterium werden dabei Grenzen aus der Literatur entnommenen, die in dem Datenspeicher 15 abgespeichert sind. Weiterhin können vom behandelnden Arzt für den Patienten individuelle Grenzen definiert werden.

Dazu kann ein Expertensystem eingesetzt werden, das die Ereignisdaten krankheits- und problemspezifisch anhand von Regelsystemen oder auf Wahrscheinlichkeiten basierend interpretiert. Dabei ist eine Individualisierung des Expertensystems auf den Patienten vorsehbar, d.h. das Expertensystem lernt den Patienten, den es überwacht, im Überwachungsprozess immer besser kennen, es trifft dazu als lernendes System ständig Prognosen über zu erwartende zukünftige Ereignisdaten, die es mit den wahren Ereignisdaten vergleicht. Damit wird eine individualisierte Überwachung realisiert.

Im Fall komplexer Alarme kann ein Expertensystem eingesetzt werden, dass die Prozesskette, Hinweise an den Patienten, Verständigung des Pflegedienstes oder Hausarzt, Rufen des Notarztes, Organisieren des Transportdienstes und/oder Vorbereitung der Klinik unter Berücksichtigung der Verfügbarkeit der Alarmempfänger übernimmt.

Die Prozesskette ist dabei die Gesamtheit der beteiligten Personen und Institutionen. Dazu gehören der Patient und seine Angehörigen, die Behandlungskette mit ambulantem Pflegedienste, Altenpfleger, Krankenschwestern, niedergelassene Ärzten, oder Klinik-Ärzten.

Alarme können in Abhängigkeit von der Dringlichkeit einer Reaktion in unterschiedlichen Dringlichkeitsstufen generiert werden, beispielsweise dringendst, dringend, Routine oder Standard. Welche Ereignisdaten zu welchen Dringlichkeitsstufen führen, kann mit den gleichen Mechanismen wie die Beurteilung der Ereignisdaten festgelegt werden und in dem Datenspeicher 15 abgespeichert sein.

Durch das erfindungsgemäße System wird eine "virtuelle" Pflegestation beim Patienten zu Hause geschaffen, die ein zentrales Alarmgebersystem aufweist, das Alarme generiert bei:
- Mangelhafter Patientencompliance (erwartete Ereignisdaten des Patienten bleiben aus)
- Überschreiten von Schwellwerten (d.h. die Ereignisdaten liegen in einem krankhaften Bereich)
- Mangelhafter Arztkompliance (erwartete Reaktion auf Alarm durch den Arzt bleibt aus).

Dieses erfindungsgemäße System zum Home Monitoring dient zur Erkennung eines medizinischen oder pflegerischen Handlungsbedarfes. Dazu werden beim Patienten die Anzahl und die Art unerwünschter Ereignisse erfasst und an das System übermittelt.

Unerwünschte Ereignisse können sein, wobei jeweils das Datum und die Uhrzeit mit erfasst werden:
- Verwirrtheitszustände
- Stürze
- Aggressivität gegen Zimmerkumpel, Pflegepersonal oder Angehörige
- Vagabundieren
- zu starke Sedierung und/oder
- typische Medikamentennebenwirkungen, die auf eine Überdosierung hinweisen.

Steigt die Anzahl der unerwünschten Ereignisse über eine Sollwertgrenze, die für jede Ereignisart unterschiedlich sein kann oder ist das unerwünschten Ereignisse gar ein "absolutes" unerwünschtes Ereignis so bewirkt das System den Aufbau einer asynchronen Kommunikation zwischen Arzt, Pflegediensten wie ambulante Pflegekräfte oder Altenpfleger, Patienten und/oder Angehörigen des Patienten.

Das erfindungsgemäße System lässt sich erfindungsgemäß beim Patienten zuhause, in Alten- oder in Pflegeheimen einsetzen.

## Patentansprüche

1. Medizinisches System zur kontinuierlichen Überwachung von alterspsychiatrischen Patienten in häuslicher Umgebung, in einem Alten- oder einem Pflegeheim mit einer Vorrichtung (2) zur Erfassung von Ereignissen bei dem Patienten wie Verwirrtheitszustände, Stürze, Aggressivität, Vagabundieren, Sedierung und/oder Medikamentennebenwirkungen, einer Vorrichtung (3 bis 8) zur Übertragung der erfassten Daten der Ereignisse an eine Systemzentrale (6), und einer Empfangsvorrichtung (20 bis 22) bei einer überwachenden Person, wobei die Systemzentrale (6) einen Speicher (15) für die Daten der erfassten Ereignisse und vorgegebenen unerwünschten Ereignissen, eine Auswertevorrichtung (12) für die Daten mit einem Komparator zum Vergleich der Daten mit den gespeicherten Sollwerten der vorgegebenen unerwünschten Ereignisse, eine Alarmvorrichtung zur Erzeugung eines Alarmsignals und eine Routingvorrichtung (14) zur Weiterleitung des Alarmsignals an eine Empfangsvorrichtung (3 bis 5, 20 bis 22) der zu alarmierenden Person einer Prozesskette aufweist, wobei die Alarmvorrichtung so ausgebildet ist, dass sie ein Alarmsignal bei Überschreitung von gespeicherten Sollwertgrenzen durch die Anzahl von unerwünschten Ereignisse sowie Ausbleiben von Reaktionen der Personen der Prozesskette erzeugt.

2. Medizinisches System nach Anspruch 1, **dadurch gekennzeichnet, dass** die Alarmvorrichtung derart ausgebildet ist, dass sie ein Alarmsignal bei Überschreitung der Daten von gespeicherten Sollwertgrenzen erzeugt, das an eine Empfangsvorrichtung (20 bis 22) einer Person einer Behandlungskette geleitet wird.

3. Medizinisches System nach Anspruch 1 oder 2, **dadurch gekennzeichnet, dass** die Alarmvorrichtung ein Alarmsignal bei signifikanter Überschreitung der Daten von gespeicherten Sollwertgrenzen erzeugt, das an eine Empfangsvorrichtung (20 bis 22) einer Person einer Notfallkette geleitet wird.

4. Medizinisches System nach einem der Ansprüche 1 bis 3, **dadurch gekennzeichnet, dass** die Alarmvorrichtung derart ausgebildet ist, dass sie ein Alarmsignal bei Ausbleiben von Daten erzeugt, das an eine Empfangsvorrichtung (3 bis 5) des Patienten geleitet wird.

5. Medizinisches System nach einem der Ansprüche 1 bis 4, **dadurch gekennzeichnet, dass** die Routingvorrichtung (14) derart ausgebildet ist, dass das Alarmsignal durch automatisches Routing an eine von der Routingvorrichtung (14) unter Berücksichtigung der Verfügbarkeit bestimmte Empfangsvorrichtung (3 bis 5, 20 bis 22) der Prozesskette geleitet wird.

6. Medizinisches System nach einem der Ansprüche 1 bis 5, **dadurch gekennzeichnet, dass** die Routingvorrichtung (14) ein lernendes Expertensystem aufweist, das die Alarmierung der Prozesskette bewirkt.

7. Medizinisches System nach einem der Ansprüche 1 bis 6, **dadurch gekennzeichnet, dass** die Auswertevorrichtung (12) ein lernendes Expertensystem aufweist, das die Daten krankheits- und/oder problemspezifisch anhand von Regelsystemen oder auf Wahrscheinlichkeiten basierend interpretiert, aufgrund deren Ergebnis das Alarmsignal ausgelöst wird.
